# EUROPÄISCHE PATENTSCHRIFT

(11) **EP 3 548 122 B1**
(45) Veröffentlichungstag und Bekanntmachung des Hinweises auf die Patenterteilung: **03.11.2021**
(21) Anmeldenummer: 17822174.3
(22) Anmeldetag: 04.12.2017
(51) Int. Cl.: A61M 5/315

(54) **KOLBEN FÜR EINE MEDIZINISCHE SPRITZE**
PLUNGER FOR A MEDICAL SYRINGE
PISTON DESTINÉ À UNE SERINGUE MÉDICALE

(30) Priorität: 02.12.2016 DE 102016123302
(43) Veröffentlichungstag der Anmeldung: 09.10.2019
(73) Patentinhaber: Datwyler Pharma Packaging Belgium NV, 3570 Alken (BE); Universiteit Hasselt, 3500 Hasselt (BE)
(72) Erfinder: THIJS, Anita, 3570 Alken (BE); JONGEN, Bram, 3512 Stevoort (BE); DEFERME, Wim, 3940 Hechtel (BE); VERBOVEN, Inge, 3470 Kortenaken (BE)
(74) Vertreter: Müller, Enno
(86) Internationale Anmeldenummer: PCT/EP2017/081326
(87) Internationale Veröffentlichungsnummer: WO 2018/100201

(56) Entgegenhaltungen:
- WO-A1-2004/044464
- WO-A1-2016/039816
- US-A1- 2014 062 036
- US-A1- 2014 319 778
- US-A1- 2014 339 777

## Beschreibung

### Gebiet der Technik

Die Erfindung betrifft einen Kolben nach den Merkmalen des Oberbegriffs des Anspruches 1.

### Stand der Technik

Kolben der in Rede stehenden Art sind bekannt. Diese finden insbesondere Verwendung in medizinischen Spritzen zur Verabreichung oder Abziehen von Flüssigkeiten an einen bzw. von einem Patienten, weiter beispielsweise, wie nachstehend auch bevorzugt, in Spritzen mit einem zylindrischen Körper aus Glas oder Kunststoff. Zwischen dem Kolben und der Wandungsinnenfläche des zylindrischen Spritzenkörpers ist eine flüssigkeitsdichte Dichtung erforderlich. Hierzu ist es bekannt, zumindest eine mit Bezug zu der Kolbenlängsachse umlaufende Dichtwulst anzuordnen. Die zumindest eine Dichtwulst ist einer vorderen Kolbenseite zugeordnet, die bei einer Verabreichung einer medizinischen Substanz mittels der Spritze der Substanz zugewandt ist und mit dieser in Kontakt steht.

Ein solcher Kolben ist beispielsweise aus der WO 2004/044464 A1 bekannt. Aus dieser Schrift ist darüber hinaus bekannt, die vordere Seite des Kolbens mit einer folienartigen Decklage abzudecken. Hierdurch wird eine Trennung des den Kolbenkörper formenden Kolbenmaterials zu der in dem Spritzenzylinder befindlichen medizinischen Substanz erreicht.

Bei einem aus der US 2014/0319778 A1 bekannten Kolben ist eine erster, zugeordnet der vorderen Seite des Kolbens ausgebildete Dichtwulst mit einem radial äußeren Zylinderabschnitt ausgebildet. Die Decklage erstreckt sich über mehrere Dichtwülse in Längsrichtung. Die Dicke im äußersten Bereich der Dichtwulst liegt zwischen 10 und 40 µm, im vordersten Bereich zwischen 20 und 50 µm.

Aus der US 2014/0339777 A1 ist ein Kolben für eine medizinische Spritze bekannt, bei welcher sich die Decklage über die erste und zweite Dichtwulst hinaus erstreckt. Beide Dichtwulste sind in einem Längsschnitt mit einem Zylinderabschnitt ausgebildet. Die Dicke der Decklage ist im Bereich von 5 bis 150 µm angegeben. Es ist jeweils eine durchgehend gleiche Dicke der Decklage gegeben.

Aus der WO 2016/039816 A1 ist ein Kolben mit einer Filmlagenbeschichtung bekannt. Die Filmlage weist eine durchgehend gleiche Dicke auf. Im Weiteren ist auch die vorderste Dichtwulst im radial äußersten Bereich mit einnem Zylinderabschnitt ausgebildet.

### Zusammenfassung der Erfindung

Ausgehend von dem dargelegten Stand der Technik beschäftigt sich der Erfindung mit der Aufgabenstellung, einen Kolben der in Rede stehenden Art bei möglichst guten Gleiteigenschaften vorteilhaft herstellbar auszubilden.

Diese Aufgabe ist beim Gegenstand des Anspruches 1 gelöst, wobei darauf abgestellt ist, dass die Decklage mit unterschiedlichen Dicken ausgebildet ist, wobei die Dicke im Bereich der Längsachse am größten ist und ausgehend von der Längsachse nach radial außen hin abnimmt, dass der Kolben mehrere sich in einer Ebene senkrecht zu der Längsachse erstreckende Dichtwulste aufweist, mit einer ersten Dichtwulst zugeordnet der vorderen Seite des Kolbens, wobei eine Dichtwulst bezogen auf einen vor einem Einsetzen des Kolbens in eine Spritze betrachteten Längsquerschnitt durch den Kolben gerundet verläuft, mit einer größten radialen Abmessung in einer zu der Längsachse senkrecht verlaufenden Ebener, dass sich die Decklage bis in den radial äußersten Bereich der ersten Dichtwulst und in axialer Richtung ausgehend von einem ersten Erreichen des äußersten Bereichs der ersten Dichtwulst in Richtung der Längsachse über 0,1 mm oder mehr erstreckt, bis hin zu 3 mm, dass die Decklage eine geringste Dicke von 0,1 bis 3 µm aufweist, dass die Decklage im zentralen Bereich der vorderen Kolbenseite eine Dicke im Bereich von 0,2 bis 60 µm aufweist, und dass die Decklage sich bis auf eine einer vorderen Seite des Kolbens abgewandte Rückseite der ersten Dichtwulst erstreckt, hierbei aber nicht einen nachfolgenden zylindrischen Abschnitt des Kolbens erreicht.

Die Decklage ist im zentralen Bereich der vorderen Kolbenseite, die zudem in Form einer Spitze ausgebildet sein kann, mit einer größeren Materialstärke aufgebracht als im radial äußeren, von der Decklage überdeckten Bereich des Kolbens. Die Dicke im Bereich der Längsachse entspricht einem Mehrfachen, beispielsweise einem 2- bis 20-Fachen oder 5- bis 10-Fachen der geringsten Dicke der Decklage im radial zu der Längsachse entfernten Bereich.

Die Decklage weist eine geringste Dicke von 0,1 bis 3 µm auf.

In weiterer Ausgestaltung kann die Decklage eine geringste Dicke von 0,4 bis 1 µm aufweisen. In bevorzugter Ausgestaltung weist die Decklage eine geringste Dicke von 0,5 µm auf.

Der Kolben kann eine oder mehrere sich jeweils in einer Ebene senkrecht zu der Längsachse erstreckende Dichtwulste aufweisen, mit einem ersten Dichtwulst zugeordnet der vorderen Seite des Kolbens, wobei die Dichtwulst bezogen auf einen vor einem Einsetzen des Kolbens in eine Spritze betrachteten Längsquerschnitt durch den Kolben gerundet verläuft, mit einer größten radialen Abmessung in einer zu der Längsachse senkrecht verlaufenden Ebene.

Im eingesetzten Zustand des Kolbens in die Spritze bzw. in den Spritzenzylinder kann die Dichtwulst im Bereich ihrer größten radialen Erstreckung dichtend an der Zylinderinnenfläche der Spritze anliegen. Hierbei kann weiter die Dichtwulst in unmittelbarem Kontaktbereich mit der Zylinderwandung ihren gerundeten Verlauf partiell verlieren, so dass es gegebenenfalls zu einer flächigen Anlage zwischen Dichtwulst und Zylinderinnenfläche kommt. Im Querschnitt können so gerundet verlaufende Abschnitte der Dichtwulst durch einen geradlinig verlaufenden Anlageabschnitt verbunden sein. So kann sich im Bereich der größten radialen Abmessung des Kolbens, weiter im Bereich der Dichtwulst, insbesondere im Bereich der der vorderen Seite des Kolbens zugeordneten Dichtwulst, ein Zylinderabschnitt bilden, der sich über 1/10 mm oder mehr bis hin zu 2 mm oder mehr in Richtung der Längsachse erstreckt.

Die Decklage erstreckt sich bis in den radial äußersten Bereich der ersten Dichtwulst und in axialer Richtung ausgehend von einem ersten Erreichen des äußersten Bereichs der ersten Dichtwulst in Richtung der Längsachse über 0,1 mm oder mehr, bis hin zu 3 mm.

In einer Anordnungsstellung des Kolbens in dem Spritzenzylinder die gesamte, bis in die Dichtzone zu dem Spritzenzylinder in Kontakt mit der medizinischen Substanz tretende Oberfläche des Kolbens von der Decklage überdeckt. Die Decklage erstreckt sich entsprechend unter Einschluss der Dichtzone, wobei in dieser Dichtzone die geringste Dicke der Decklage erreicht sein kann. Das Decklagenmaterial kann so in vorteilhafter Weise zusätzlich zu günstigen Gleiteigenschaften des Kolbens an der Zylinderwandungs-Innenfläche beitragen.

Die Dicke der Decklage kann nach radial außen kontinuierlich oder stufenartig abnehmen. Bei einer stufenartigen Abnahme kann nicht nur eine sprunghafte Dickenabnahme des Decklagenmaterials gegeben sein, sondern vielmehr auch eine in diesem Stufenbereich gegebene kontinuierliche Dickenabnahme, die jedoch im Querschnitt des Kolbens betrachtet über ein Radialerstreckungsmaß eine größere Dickenabnahme aufweist als in den Decklagenbereichen vor und hinter der Stufe in einem gleichbetrachteten Radialerstreckungsmaß. Auch kann ausgehend von dem Bereich größter Dicke mit zunehmender radialer Beabstandung zunächst die Dicke der Decklage gleichbleiben und erst mit Näherung an den radial äußeren Bereich abnehmen.

Die geringste Dicke der Decklage kann dem radial äußersten Bereich des Kolbens im Bereich der ersten Dichtwulst zugeordnet sein. Über diesen radial äußersten Bereich hinaus - betrachtet ausgehend von der vorderen Seite des Kolbens - kann die Decklage, sofern vorhanden, bezüglich ihrer Dicke wiederum stärker gewählt sein, alternativ die geringste Dicke gleichbleibend aufweisen oder sogar bis auf Null abnehmen.

Eine radiale Abmessung der Decklage ausgehend von einem Radialmaß, das bei einem ersten Unterschreiten eines 3-Fachen der geringsten Dicke gegeben ist, kann einem Fünftel oder weniger, beispielsweise einem Sechstel oder einem Zehntel, bis hin zu einem zwanzigstel, des größten radialen Maßes entsprechen.

Die Decklage kann im Sprühverfahren auf den Kolben aufgebracht sein. Dieses Sprühverfahren erfolgt bevorzugt nach einer Herstellung des beispielsweise aus einem Gummimaterial bestehenden Kolbenkörpers.

Auch kann die Decklage als gesondertes Teil ausgebildet sein, das auf den Kolben aufgebracht ist. Es kann sich hierbei um eine vorgefertigte Folie handeln, die in bevorzugter Ausgestaltung eine von innen nach außen abnehmende Materialstärke aufweist.

Die Aufbringung der folienartigen Decklage auf den Kolben kann zufolge einer Verschmelzung erreicht sein. Darüber hinaus ist auch die komplette Herstellung des Kolbens mit der folienartigen Decklage im In-Mold-Labeling-Verfahren möglich.

Auch ist eine Herstellung des mit der Decklage versehenen Kolbens im Zwei-Komponenten-Spritzverfahren möglich.

Die Decklage kann auf ihrer dem Kolben zugewandten Seite einen oder mehrere Vorsprünge aufweist, für eine Formschlusseinpassung in entsprechende Ausnehmungen in der vorderen Seite des Kolbens. Hierdurch kann eine lageorientierte Zuordnung der Decklage, insbesondere wenn diese als gesondertes Teil hergestellt ist, erreichbar sein. Die kolbenseitige Ausnehmung ist bevorzugt formschlussangepasst an den Vorsprung der Decklage.

So kann bezogen auf einen Längsquerschnitt durch den Kolben ein Vorsprung und eine zugeordnete Ausnehmung symmetrisch angeordnet sein oder von der Längsachse mittig durchsetzt sein. Bevorzugt ist diesbezüglich eine zur Längsachse des Kolbens konzentrische Anordnung von Ausnehmung und Vorsprung.

Der Kolben kann über seine gesamte Seitenfläche, mit Ausnahme bis zum Bereich der ersten Dichtwulst, eine Dichtlage konstanter Dicke aufweisen. Entsprechend kann, betrachtet von der vorderen Seite des Kolbens, nach Erreichen der ersten radialen Größterstreckung im Bereich der ersten Dichtwulst die Decklagendicke gleichbleibend bis hin zur rückwärtigen Seite des Kolbens gegeben sein.

Die konstante Dicke, die im Bereich der gesamten Seitenfläche gegeben sein kann, kann 0,3 bis 3 µm betragen, weiter beispielsweise 0,4 bis 1 µm, insbesondere 0,5 µm. Darüber hinaus kann die konstante Dicke im Bereich der gesamten Seitenfläche der geringsten Decklagendicke entsprechen.

Der dünnste Bereich der Decklage ist in bevorzugter Ausgestaltung maßlich kleiner gewählt als es der Oberflächen-Rautiefe des Kolbenmaterials entspricht. Es kann sich bei einem Auftrag der Decklage im Sprühverfahren eine dachartige Überspannung der sich zufolge Rauigkeit ergebenen Täler in der Kolbenoberfläche einstellen.

Die Decklage kann, beispielsweise in Form eines aufzubringenden folienartigen gesonderten Teils, aus einem fluorbasierten Kunststoffmaterial bestehen, beispielsweise aus einem fluorhaltigem (Co-)Polymer, so weiter beispielsweise Ethylen-Tetrafluorethylen (ETFE), Perfluorethylenpropylen-Copoylmer (FEP), ein Terpolymer von Tetrafluorethylen, Hexafluorpropylen und Vinylidenfluorid (THV), Polyvinylfluorid (PVF), Perfluoralkoxy-Polymer (PFA), spritzgießfähiges Polytetrafluorethylen (PTFE), Polychlortrifluorethylen (PCTFE), Ethylenchlortrifluorethylen (ECTFE) oder Polyvinylidenfluorid (PVDF).

Die vor- und nachstehend angegebenen Bereiche bzw. Wertebereiche oder Mehrfachbereiche schließen hinsichtlich der Offenbarung auch sämtliche Zwischenwerte ein, insbesondere in 1/10-Schritten der jeweiligen Dimension, gegebenenfalls also auch dimensionslos. Beispielsweise beinhaltet die Angabe 0,3 bis 3 µm auch die Offenbarung von 0,4 bis 3 µm, 0,3 bis 2,9 µm, 0,4 bis 2,9 µm, etc. Diese Offenbarung kann einerseits zur Eingrenzung einer genannten Bereichsgrenze von unten und/oder oben, alternativ oder ergänzend aber zur Offenbarung eines oder mehrerer singulärer Werte aus einem jeweilig angegebenen Bereich dienen.

### Kurze Beschreibung der Zeichnungen

Nachstehend ist die Erfindung anhand der beigefügten Zeichnung erläutert, die aber lediglich Ausführungsbeispiele darstellt. Ein Teil, der nur bezogen auf eines der Ausführungsbeispiele erläutert ist und bei einem weiteren Ausführungsbeispiel aufgrund der dort herausgestellten Besonderheit nicht durch ein anderes Teil ersetzt ist, ist damit auch für dieses weitere Ausführungsbeispiel als jedenfalls mögliches vorhandenes Teil beschrieben. Auf der Zeichnung zeigt:
- Fig. 1: in partieller Schnittdarstellung eine medizinische Spritze mit einem Kolben;
- Fig. 2: einen Kolben einer ersten Ausführungsform in Ansicht im Zuge eines Aufsprühvorganges zur Aufbringung einer Decklage;
- Fig. 3: den mit der Decklage gemäß Figur 3 versehenen Kolben im Vollschnitt;
- Fig. 4: die Herausvergrößerung des Bereiches IV in Figur 3;
- Fig. 5: eine makroskopische Vergrößerung des Bereiches V in Figur 4;
- Fig. 6: eine Darstellung gemäß Figur 2, betreffend eine zweite Ausführungsform bei Aufbringen der Decklage als gesonderte Folienlage;
- Fig. 7: eine der Figur 3 entsprechende Darstellung, die Ausführungsform gemäß Figur 6 betreffend;
- Fig. 8: die Herausvergrößerung des Bereiches VIII in Figur 7;
- Fig. 9: betreffend welche nicht Teil der vorliegenden eine der Figur 3 entsprechende Darstellung, eine weitere Ausführungsform Erfindung darstellt.
- Fig. 10: in einer weiteren Ausführungsform den Kolben in einer Darstellung gemäß Figur 3,
- Fig. 11: in einer Herausvergrößerung gemäß Figur 4 eine weitere Ausführungsform;
- Fig. 12: ein weiteres Ausführungsbeispiel in einer Darstellung gemäß Figur 4.

### Beschreibung der Ausführungsformen

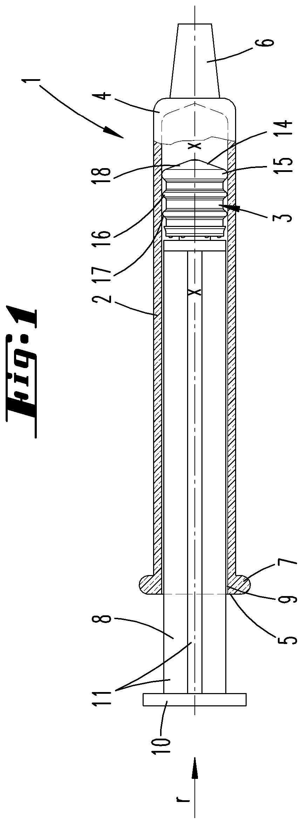

Dargestellt und beschrieben ist, zunächst mit Bezug zu Figur 1, eine Spritze 1 für medizinische Zwecke.

Die Spritze 1 setzt sich im Wesentlichen zusammen aus einem langgestreckten, beidseitig offenen Zylinder 2, insbesondere einem zylindrischen Körper, beispielsweise aus Glas oder Kunststoff, und einem in dem Zylinder 2 in Vorschubrichtung r bzw. entgegen Vorschubrichtung r verlagerbaren Kolben 3.

Der Zylinder 2 besitzt ein distales Ende 4 und ein proximales Ende 5. Im Bereich des distalen Endes 4 geht der Zylinder 2 über in einen spitzenförmigen Nadelansatz 6.

Im Bereich des proximalen Endes 5 ist an dem Zylinder 2 eine Radialerweiterung in Form eines Flansches 7 angeformt. Dieser dient im Zuge der Nutzung der Spritze 1 als Gegenhalter zum Untergriff bevorzugt durch Zeige- und Mittelfinger der die Spritze 1 bedienenden Hand.

Der Kolben 3 ist verbunden mit einer Kolbenstange 8. Diese durchsetzt eine im proximalen Ende 5 vorgesehene Einführöffnung 9 des Zylinders 2 und formt im Bereich des freien, dem Kolben 3 abgewandten Endes eine quer zur Längserstreckung der Kolbenstange 8 ausgerichtete, plattenartige Daumenauflage 10.

Die Kolbenstange 8 kann, wie dargestellt, in Form kreuzartig angeordneter Stege 11 ausgebildet sein.

Der Kolben der dargestellten Ausführungsformen ist bevorzugt aus einem Gummimaterial hergestellt.

Weiter weist der Kolben 3 eine Längsachse x auf, welche im Nutzungszustand gemäß Figur 1 mit der Längsachse des Zylinders 2 der Spritze 1 zusammenfällt.

In Längserstreckungsrichtung vom mit Bezug zu der Vorschubrichtung r hinteren Ende aus zugänglich, ist an dem Kolben 3 koaxial zur Längsachse x eine Aufnahmeöffnung 12 mit einem Innengewinde 13 ausgebildet, zur Schraubfestlegung des zugewandten distalen Endes der Kolbenstange 8.

Der Kolben 3 ist in Nutzungsstellung dem distalen Ende 4 des Zylinders 2 zugewandt im Bereich der vorderen Seite des Kolbens 3 mit einem Spitzenabschnitt 14 versehen. Dieser Spitzenabschnitt 14 kann gemäß den Ausführungsbeispielen kegelförmig gestaltet sein. Durch den gegebenen Spitzenabschnitt 14 ist ein vollständiges Ausdrücken von Flüssigkeit aus dem Inneren des Zylinders 2 über den koaxial zur Längsachse x im Bereich des distalen Endes 4 angeordneten Nadelansatz 6 ermöglicht.

Der Kolben 3 ist zumindest mit einer mit Bezug zur Längsachse x umlaufenden Dichtwulst 15 versehen. In den dargestellten Ausführungsbeispielen sind drei solcher Dichtwulste 15 bis 17 vorgesehen, die zueinander in Erstreckungsrichtung der Längsachse x beabstandet, bevorzugt gleichmäßig zueinander beabstandet sind.

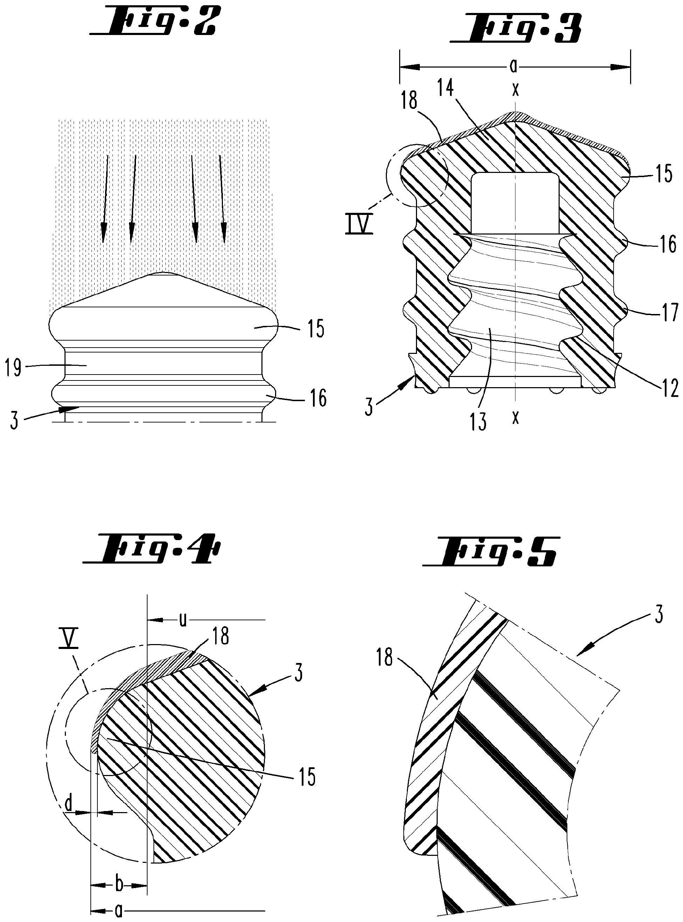

Die Dichtwulst 15 ist hierbei der vorderen Seite des Kolbens 3 zugeordnet, schließt in bevorzugter Ausgestaltung unmittelbar an den Spitzenabschnitt 14 an, so dass die im Querschnitt gemäß Figur 3 dachartig ausgerichtete Fläche des Spitzenabschnitts 14 bevorzugt stufenfrei übergeht in die Oberfläche der Dichtwulst 15.

Die Dichtwulste 15, sowie gegebenenfalls 16 und 17 sind insbesondere vor Einsatz des Kolbens 3 in den Zylinder 2 im Querschnitt gerundet verlaufend ausgebildet. Es ergeben sich entsprechend ringwulstartige Dichtzonen, die untereinander verbunden sind über bevorzugt rein zylindrische Kolbenabschnitte.

Die Dichtwulste 15 bis 17 dienen zur flüssigkeitsdichten Abstützung des Kolbens 3 an der Zylinder-Innenwandung.

Jeder Dichtwulst, insbesondere aber die Dichtwulst 15 besitzt eine größte radiale Abmessung a in einer senkrecht zur Längsachse x verlaufenden Ebene, welche größte radiale Abmessung a bei unbeeinflusstem Kolben 3, d. h. bei nicht in den Zylinder 2 eingesetztem Kolben 3, im Zenit der verrundeten Dichtwulst 5 sich ergibt.

Der Kolben 3 ist im Bereich seiner vorderen Seite von einer Decklage 18 überdeckt. Hierbei kann es sich um eine Lage handeln, die die der medizinischen Substanz zugewandte Oberfläche des Kolbens 3 bei Nutzung der Spritze 1 versiegelt, so dass kein unmittelbarer Kontakt zwischen dem eigentlichen Kolbenmaterial und der medizinischen Substanz zustande kommt.

Wie aus den Darstellungen ersichtlich, erstreckt sich mit Bezug zu einem Querschnitt, beispielsweise gemäß Figur 3, die Decklage 18 ausgehend von einem mittigen, von der Längsachse x durchsetzten Spitzenbereich des Kolbens 3 nach radial außen zumindest bis zum Erreichen der größten radialen Abmessung a im Bereich der ersten Dichtwulst 15.

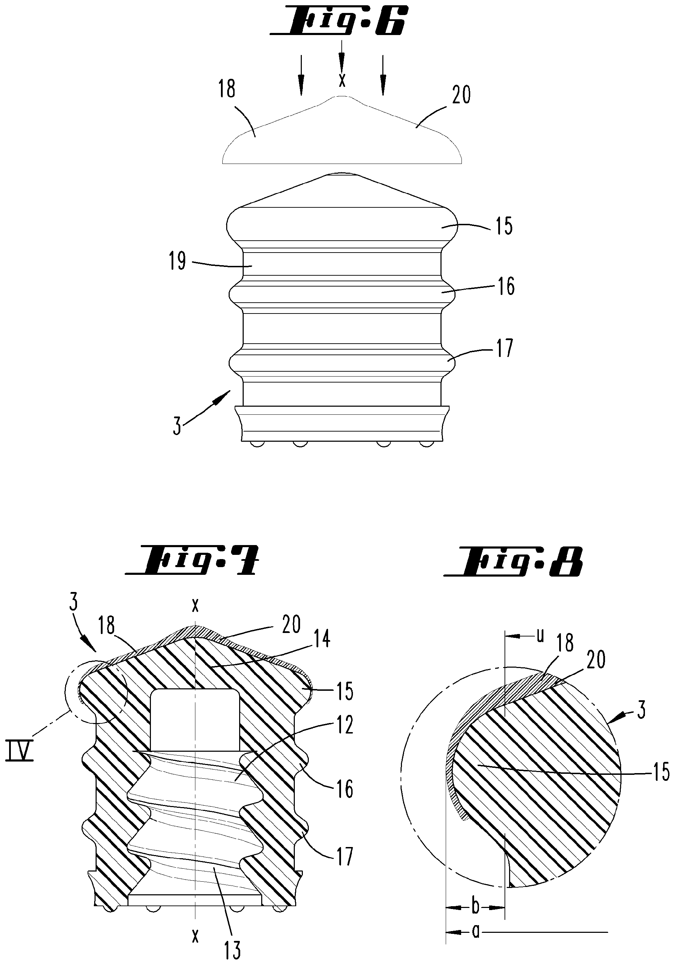

Gemäß der Ausführungsform in Figur 7 kann die Decklage 18 sich auch ausgehend von der vorderen Seite des Kolbens 3 über die radiale Abmessung a im Bereich der Dichtwulst 15 hinaus erstrecken, dies ausgehend von der Ebene der größten radialen Abmessung a in Erstreckungsrichtung der Längsachse x über ein Maß von 2 µm oder mehr bis hin zu 3 mm oder mehr. Dargestellt ist eine Ausführungsform, bei welcher sich die Decklage 18 bis auf die der vorderen Seite des Kolbens 3 abgewandte Rückseite der ersten Dichtwulst 15 erstreckt, hierbei nicht den nachfolgenden zylindrischen Abschnitt des Kolbens 3 erreicht.

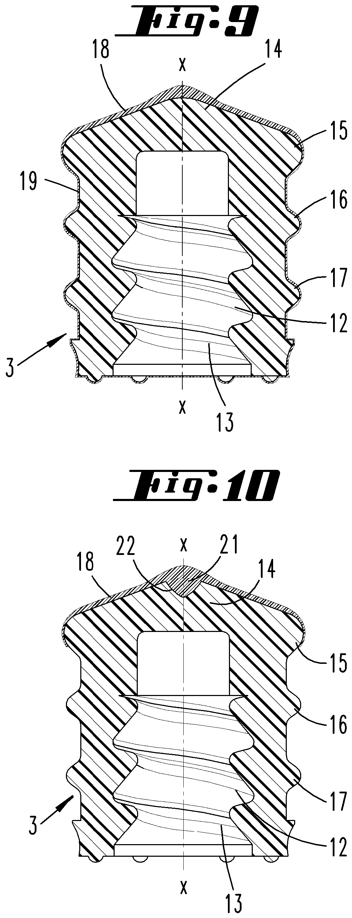

Auch kann gemäß der Darstellung in Figur 9 die Decklage 18 die vollständige Seitenfläche 19 des Kolbens 3 überdeckend vorgesehen sein. Die Decklage erstreckt sich hier über den Spitzenabschnitt 14 und seitlich des Kolbens 3 über alle Dichtwulste 15 bis 17 sowie die diese verbindenden Zylinderabschnitte des Kolbens 3 bis hin zum rückwärtigen Ende des Kolbens 3.

Wie weiter aus den Darstellung zu erkennen, ist ausgehend vom durch die Längsachse x durchsetzten Bereich nach radial außen eine abnehmende Dicke d der Decklage 18 vorgesehen. Die Abnahme der Dicke von innen nach radial außen kann, wie dargestellt, kontinuierlich vorgesehen sein.

Im Bereich der größten radialen Abmessung a, d. h. bevorzugt im Zenitbereich der ersten, der vorderen Seite des Kolbens 3 zugeordneten Dichtwulst 15, kann die geringste Dicke der Decklage 18 vorgesehen sein. Die geringste Dicke d der Decklage 18 kann beispielsweise 0,5 µm betragen.

Es kann sich so eine Decklage 18 ergeben, bei welcher eine radiale Abmessung b der Decklage 18 ausgehend von einem Radialmaß u einem Fünftel oder weniger der größten radialen Abmessung a entspricht, wobei das Radialmaß u gegeben ist bei einem ersten Unterschreiten eines 3-Fachen der geringsten Dicke d der Decklage 18 (vgl. Figur 3).

Die Decklage 18 kann durch Aufsprühen auf die Kolbenvorderseite aufgebracht sein (vgl. hierzu Figuren 2 bis 5).

Hierbei ergibt sich insbesondere im gekrümmten Abschnitt der Dichtwulst 15, weiter im Bereich der größten radialen Abmessung a eine Verringerung der Decklagendicke d.

Die geringste Dicke der Decklage weist hierbei ein Maß auf, welches bevorzugt kleiner gewählt ist, als es der Rautiefe des Kolbenmaterials entspricht.

Auch kann die Decklage 18 als gesondertes Teil 20, insbesondere als Folienteil ausgebildet sein (vgl. Figur 6). Dieses gesonderte Teil wird gesondert auf die vordere Seite des Kolbens 3 aufgebracht und an diesem verhaftet oder, alternativ, im Zuge der Herstellung des Kolbens 3 beispielsweise durch ein In-Mold-Verfahren mit dem Kolbenmaterial verbunden.

Die folienartige Decklage 18 kann im Zuge des diesbezüglichen Herstellungsprozesses mit einer nach radial außen abnehmenden Dicke versehen sein, bis hin zu einer geringsten Dicke im bevorzugt radial äußeren Bereich. Mit Erreichen der bevorzugt vorgegebenen geringsten Dicke erstreckt sich das Lagenmaterial weiter unter Einhaltung dieser geringsten Dicke.

Insbesondere ein solches gesondert gefertigtes Teil 20 kann zur exakten Ausrichtung an dem Kolben 3 mit einem in Richtung des Kolbens 3 weisenden Vorsprung 21 versehen sein, der in eine zugeordnete Ausnehmung 22 des Kolbens 3, beispielsweise im Bereich seines Spitzenabschnittes 14 zur Formschlusseinpassung einsetzbar ist (vgl. Figur 10).

Auch kann eine Kombination von Aufsprühen einer Decklage und Aufbringen einer gesondert hergestellten Decklage vorgesehen sein, so weiter beispielsweise bei einer möglichen Ausgestaltung des Kolbens 3 gemäß der Darstellung in Figur 9, bei welcher auch der umlaufende Kolbenmantel mit einer Decklage 18 versehen ist. Diese Decklage kann in gleichmäßiger Dicke aufgesprüht sein, hierbei bis in den Bereich des kolbenseitigen Spitzenabschnittes 14, weiter diesen gegebenenfalls vollständig überdeckend. Abschließend kann auf den Spitzenabschnitt 14, entsprechend auf der vorderen Seite des Kolbens 3, eine mit einer nach radial außen abnehmenden Dicke versehene Decklagen-Folie aufgebracht sein.

Das Material der Decklagen 18 zur Überdeckung der Kolben-Seitenfläche 19 kann gleich oder auch unterschiedlich zu dem Decklagenmaterial des Spitzenabschnitts 14 gewählt sein.

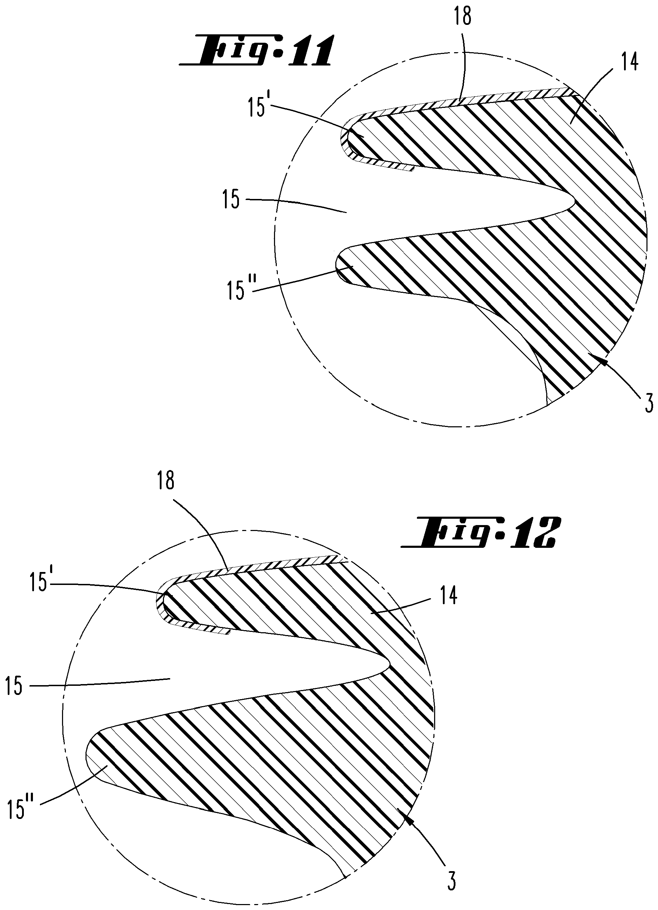

Wie in den vergrößerten Darstellungen in den Figuren 11 und 12 gezeigt, kann die Dichtwulst 15 auch als Doppelwulst mit zwei in Achsrichtung unmittelbar aufeinanderfolgenden Dichtzonen ausgebildet sein, wobei die Dichtzonen 15' und 15" gleiche Durchmesser aufweisen können. Alternativ ist die zum Spitzenbereich mehr distanzierte Dichtzone 15" im Durchmesser größer gewählt als die weniger distanzierte Dichtzone 15'. Die Decklage 18 des Spitzenabschnitts 14 umgreift hierbei bevorzugt nur die erste Dichtzone 15' der Dichtwulst 15. Die zweite Dichtzone 15" kann, wie bevorzugt, decklagenfrei unmittelbar mit der zugewandten Innenfläche des Zylinders 2 zusammenwirken.

Einen Kolben 3, der dadurch gekennzeichnet ist, dass der Kolben 3 eine oder mehrere sich in einer Ebene senkrecht zu der Längsachse x erstreckende Dichtwulste 15-17 aufweist, mit einer ersten Dichtwulst 15 zugeordnet der vorderen Seite des Kolbens 3, wobei eine Dichtwulst 15-17 bezogen auf einen vor einem Einsetzen des Kolbens 3 in eine Spritze 1 betrachteten Längsquerschnitt durch den Kolben 3 gerundet verläuft, mit einer größten radialen Abmessung a in einer zu der Längsachse x senkrecht verlaufenden Ebene.

Einen Kolben 3, der dadurch gekennzeichnet ist, dass sich die Decklage 18 bis in den radial äußersten Bereich der ersten Dichtwulst 15 und in axialer Richtung ausgehend von einem ersten Erreichen des äußersten Bereichs der ersten Dichtwulst 15 in Richtung der Längsachse x über 0,1 mm oder mehr erstreckt, bis hin zu 3 mm oder mehr.

Einen Kolben 3, der dadurch gekennzeichnet ist, dass die Dicke der Decklage 18 nach radial außen kontinuierlich oder stufenartig abnimmt.

Einen Kolben 3, der dadurch gekennzeichnet ist, dass die geringste Dicke d der Decklage 18 dem radial äußersten Bereich des Kolbens 3 im Bereich des ersten Dichtwulstes 15 zugeordnet ist.

Einen Kolben 3, der dadurch gekennzeichnet ist, dass eine radiale Abmessung a der Decklage 18 ausgehend von einem Radialmaß u, das bei einem ersten Unterschreiten eines 3-Fachen der geringsten Dicke d gegeben ist, einem Fünftel oder weniger der größten radialen Abmessung a entspricht.

Einen Kolben 3, der dadurch gekennzeichnet ist, dass die Decklage 18 im Sprühverfahren auf den Kolben 3 aufgebracht ist.

Einen Kolben 3, der dadurch gekennzeichnet ist, dass die Decklage 18 als gesondertes Teil 20 ausgebildet ist und auf den Kolben 3 aufgebracht ist.

Einen Kolben 3, der dadurch gekennzeichnet ist, dass die Decklage 18 auf ihrer dem Kolben 3 zugewandten Seite einen oder mehrere Vorsprünge 21 aufweist, für eine Formschlusseinpassung in entsprechende Ausnehmungen 22 in der vorderen Seite des Kolbens 3.

Einen Kolben 3, der dadurch gekennzeichnet ist, dass bezogen auf einen Längsquerschnitt durch den Kolben 3 ein Vorsprung 21 und eine zugeordnete Ausnehmung 22 symmetrisch angeordnet oder von der Längsachse x mittig durchsetzt sind.

Einen Kolben 3, der dadurch gekennzeichnet ist, dass der Kolben 3 über seine gesamte Seitenfläche 19, mit Ausnahme bis zum Bereich der ersten Dichtwulst 15, eine Decklage 18 konstanter Dicke d aufweist.

Einen Kolben 3, der dadurch gekennzeichnet ist, dass die konstante Dicke d 0,3 bis 3 µm beträgt.

Die Erfindung wird durch die angehängten Ansprüche definiert.

### Liste der Bezugszeichen

| | | | |
|---|---|---|---|
| 1 | Spritze | a | radiale Abmessung |
| 2 | Zylinder | b | radiale Abmessung |
| 3 | Kolben | d | Dicke |
| 4 | distales Ende | r | Vorschubrichtung |
| 5 | proximales Ende | u | Radialmaß |
| 6 | Nadelansatz | x | Längsachse |
| 7 | Flansch | | |
| 8 | Kolbenstange | | |
| 9 | Einführöffnung | | |
| 10 | Daumenauflage | | |
| 11 | Steg | | |
| 12 | Aufnahmeöffnung | | |
| 13 | Innengewinde | | |
| 14 | Spitzenabschnitt | | |
| 15 | Dichtwulst | | |
| 15' | Dichtzone | | |
| 15" | Dichtzone | | |
| 16 | Dichtwulst | | |
| 17 | Dichtwulst | | |
| 18 | Decklage | | |
| 19 | Seitenfläche | | |
| 20 | Teil | | |
| 21 | Vorsprung | | |
| 22 | Ausnehmung | | |

## Patentansprüche

1. Kolben (3) für eine medizinische Spritze (1), wobei der Kolben (3) einen Kolbenkörper mit einer Längsachse (x) aufweist, mit einer Mantelfläche, mit einer vorderen Seite und mit einer hinteren Seite, wobei der vorderen Seite des Kolbens (3) eine erste Dichtwulst (15) zugeordnet ist und die rückwärtige Seite zum Verbinden mit einer Kolbenstange (8) dient, wobei weiter ein vorderer Teil des Kolbens (3) einschließlich der vorderen Seite zur Trennung des Kolbens (3) im Übrigen von der medizinischen Substanz in der Gebrauchsstellung mit einer folienartige Decklage (18) abgedeckt ist, **dadurch gekennzeichnet, dass** die Decklage (18) mit unterschiedlichen Dicken (d) ausgebildet ist, wobei die Dicke (d) im Bereich der Längsachse (x) am größten ist und ausgehend von der Längsachse (x) nach radial außen hin abnimmt, dass der Kolben (3) mehrere sich in einer Ebene senkrecht zu der Längsachse (x) erstreckende Dichtwulste (15 bis 17) aufweist, mit einer ersten Dichtwulst (15) zugeordnet der vorderen Seite des Kolbens (3), wobei eine Dichtwulst (15 bis 17) bezogen auf einen vor einem Einsetzen des Kolbens (3) in eine Spritze (1) betrachteten Längsquerschnitt durch den Kolben (3) gerundet verläuft, mit einer größten radialen Abmessung (a) in einer zu der Längsachse (x) senkrecht verlaufenden Ebener, dass sich die Decklage (18) bis in den radial äußersten Bereich der ersten Dichtwulst (15) und in axialer Richtung ausgehend von einem ersten Erreichen des äußersten Bereichs der ersten Dichtwulst (15) in Richtung der Längsachse (x) über 0,1 mm oder mehr erstreckt, bis hin zu 3 mm, dass die Decklage (18) eine geringste Dicke (d) von 0,1 bis 3 µm aufweist, dass die Decklage (18) im zentralen Bereich der vorderen Kolbenseite eine Dicke im Bereich von 0,2 bis 60 µm aufweist, und dass die Decklage sich bis auf eine einer vorderen Seite des Kolbens abgewandte Rückseite der ersten Dichtwulst erstreckt, hierbei aber nicht einen nachfolgenden zylindrischen Abschnitt des Kolbens erreicht.

2. Kolben (3) nach Anspruch 1, **dadurch gekennzeichnet, dass** die Decklage (18) eine geringste Dicke (d) von 0,4 bis 1 µm aufweist.

3. Kolben (3) nach einem der vorhergehenden Ansprüche, **dadurch gekennzeichnet, dass** die Dicke der Decklage (18) nach radial außen kontinuierlich oder stufenartig abnimmt.

4. Kolben (3) nach einem der vorhergehenden Ansprüche, **dadurch gekennzeichnet, dass** die geringste Dicke (d) der Decklage (18) dem radial äußersten Bereich des Kolbens (3) im Bereich der ersten Dichtwulst (15) zugeordnet ist.

5. Kolben (3) nach einem der vorhergehenden Ansprüche, **dadurch gekennzeichnet, dass** eine radiale Abmessung (a) der Decklage (18) ausgehend von einem Radialmaß (u), das bei einem ersten Unterschreiten eines 3-Fachen der geringsten Dicke (d) gegeben ist, einem Fünftel oder weniger der größten radialen Abmessung (a) entspricht.

6. Kolben (3) nach einem der vorhergehenden Ansprüche, **dadurch gekennzeichnet, dass** die Decklage (18) im Sprühverfahren auf den Kolben (3) aufgebracht ist.

7. Kolben (3) nach einem der Ansprüche 1 bis 6, **dadurch gekennzeichnet, dass** die Decklage (18) als gesondertes Teil (20) ausgebildet ist und auf den Kolben (3) aufgebracht ist.

8. Kolben (3) nach einem der vorhergehenden Ansprüche, **dadurch gekennzeichnet, dass** die Decklage (18) auf ihrer dem Kolben (3) zugewandten Seite einen oder mehrere Vorsprünge (21) aufweist, für eine Formschlusseinpassung in entsprechende Ausnehmungen (22) in der vorderen Seite des Kolbens (3).

9. Kolben (3) nach Anspruch 8, **dadurch gekennzeichnet, dass** bezogen auf einen Längsquerschnitt durch den Kolben (3) ein Vorsprung (21) und eine zugeordnete Ausnehmung (22) symmetrisch angeordnet oder von der Längsachse (x) mittig durchsetzt sind.

10. Kolben (3) nach einem der vorhergehenden Ansprüche, **dadurch gekennzeichnet, dass** der Kolben (3) über seine gesamte Seitenfläche (19), mit Ausnahme bis zum Bereich der ersten Dichtwulst (15), eine Decklage (18) konstanter Dicke (d) aufweist.

11. Kolben (3) nach Anspruch 10, **dadurch gekennzeichnet, dass** die konstante Dicke (d) 0,3 bis 3 µm beträgt.

## Claims

1. Plunger (3) for a medical syringe (1), the plunger (3) having a plunger body which has a longitudinal axis (x), a lateral surface, a front side and a rear side, a first sealing bead (15) being associated with the front side of the plunger (3) and the rear side being used to connect to a plunger rod (8), a front part of the plunger (3), including the front side, furthermore being covered with a film-like cover layer (18) for also separating the plunger (3) from the medical substance in the use position, **characterised in that** the cover layer (18) is formed having different thicknesses (d), the thickness (d) being greatest in the region of the longitudinal axis (x) and, starting from the longitudinal axis (x), decreasing radially outwards, **in that** the plunger (3) has a plurality of sealing beads (15 to 17) extending in a plane perpendicular to the longitudinal axis (x), having a first sealing bead (15) which is associated with the front side of the plunger (3), a sealing bead (15 to 17) extending in a rounded manner relative to a longitudinal cross-section through the plunger (3) viewed prior to the insertion of the plunger (3) into a syringe (1), having a largest radial dimension (a) in a plane extending perpendicularly to the longitudinal axis (x), **in that** the cover layer (18) extends as far as the radially outermost region of the first sealing bead (15) and, in the axial direction, starting from a point at which the outermost region of the first sealing bead (15) is first reached, extends over 0.1 mm or more, up to 3 mm, in the direction of the longitudinal axis (x), **in that** the cover layer (18) has a smallest thickness (d) of 0.1 to 3 µm, **in that** the cover layer (18) has a thickness in the range of 0.2 to 60 µm in the central region of the front plunger side, and **in that** the cover layer extends as far as a rear side of the first sealing bead that faces away from a front side of the plunger, but does not reach a subsequent cylindrical portion of the plunger.

2. Plunger (3) according to claim 1, **characterised in that** the cover layer (18) has a smallest thickness (d) of 0.4 to 1 µm.

3. Plunger (3) according to either of the preceding claims, **characterised in that** the thickness of the cover layer (18) decreases continuously or in steps radially outwards.

4. Plunger (3) according to any of the preceding claims, **characterised in that** the smallest thickness (d) of the cover layer (18) is associated with the radially outermost region of the plunger (3) in the region of the first sealing bead (15).

5. Plunger (3) according to any of the preceding claims, **characterised in that** a radial dimension (a) of the cover layer (18) corresponds to a fifth of the largest radial dimension (a) or less, starting from a radial dimension (u) which is present when the thickness is less than 3 times the smallest thickness (d).

6. Plunger (3) according to any of the preceding claims, **characterised in that** the cover layer (18) is applied to the plunger (3) in a spraying process.

7. Plunger (3) according to any of claims 1 to 6, **characterised in that** the cover layer (18) is designed as a separate part (20) and is applied to the plunger (3).

8. Plunger (3) according to any of the preceding claims, **characterised in that** the cover layer (18) has one or more projections (21) on the side thereof facing the plunger (3), for form-fitting engagement in corresponding recesses (22) in the front side of the plunger (3).

9. Plunger (3) according to claim 8, **characterised in that**, based on a longitudinal cross section through the plunger (3), a projection (21) and an associated recess (22) are arranged symmetrically or are passed through centrally by the longitudinal axis (x).

10. Plunger (3) according to any of the preceding claims, **characterised in that** the plunger (3) has a cover layer (18) having a constant thickness (d) over the entire lateral surface (19) thereof, with the exception of up to the region of the first sealing bead (15).

11. Plunger (3) according to claim 10, **characterised in that** the constant thickness (d) is 0.3 to 3 µm.

## Revendications

1. Piston (3) pour une seringue médicale (1), ledit piston (3) comprenant un corps de piston ayant un axe longitudinal (x), une surface d'enveloppe, un côté avant et un côté arrière, dans lequel un premier bourrelet d'étanchéité (15) est associé au côté avant du piston (3) et le côté arrière sert au raccordement à une tige de piston (8), dans lequel, en outre, une partie avant du piston (3) comprenant le côté avant est recouverte d'une couche de couverture de type feuille (18) pour séparer le reste du piston (3) de la substance médicale dans la position d'utilisation, **caractérisé en ce que** la couche de couverture (18) est formée avec différentes épaisseurs (d), l'épaisseur (d) étant la plus grande dans la zone de l'axe longitudinal (x) et diminuant radialement vers l'extérieur à partir de l'axe longitudinal (x), **en ce que** le piston (3) présente une pluralité de bourrelets d'étanchéité (15 à 17) s'étendant dans un plan perpendiculaire à l'axe longitudinal (x), un premier bourrelet d'étanchéité (15) étant associé au côté avant du piston (3), dans lequel un bourrelet d'étanchéité (15 à 17) s'étend de manière arrondie par rapport à une section transversale longitudinale à travers le piston (3) considéré avant l'insertion du piston (3) dans une seringue (1), avec une dimension radiale maximale (a) dans un plan s'étendant perpendiculairement à l'axe longitudinal (x), **en ce que** la couche de couverture (18) s'étend jusque dans la région radialement la plus extérieure du premier bourrelet d'étanchéité (15) et sur 0,1 mm ou plus et jusqu'à 3 mm dans la direction axiale en partant d'une première arrivée à la région la plus extérieure du premier bourrelet d'étanchéité (15) dans la direction de l'axe longitudinal (x), **en ce que** la couche de couverture (18) a une épaisseur minimale (d) de 0,1 à 3 µm, **en ce que** la couche de couverture (18) a une épaisseur dans la plage de 0,2 à 60 µm dans la zone centrale du côté avant du piston, et **en ce que** la couche de couverture s'étend jusqu'à un côté arrière du premier bourrelet d'étanchéité opposé au côté avant du piston mais sans atteindre une partie cylindrique consécutive du piston.

2. Piston (3) selon la revendication 1, **caractérisé en ce que** la couche de couverture (18) a une épaisseur minimale (d) de 0,4 à 1 µm.

3. Piston (3) selon l'une des revendications précédentes, **caractérisé en ce que** l'épaisseur de la couche de couverture (18) diminue de manière continue ou par paliers vers l'extérieur radialement.

4. Piston (3) selon l'une des revendications précédentes, **caractérisé en ce que** l'épaisseur minimale (d) de la couche de couverture (18) est associée à la zone radialement la plus extérieure du piston (3) dans la région du premier bourrelet d'étanchéité (15).

5. Piston (3) selon l'une des revendications précédentes, **caractérisé en ce qu'**une dimension radiale (a) de la couche de couverture (18) à partir d'une dimension radiale (u) qui est donnée par le premier passage sous trois fois l'épaisseur minimale (d), correspond à un cinquième ou moins de la dimension radiale maximale (a).

6. Piston (3) selon l'une des revendications précédentes, **caractérisé en ce que** la couche de couverture (18) est appliquée sur le piston (3) par pulvérisation.

7. Piston (3) selon l'une des revendications 1 à 6, **caractérisé en ce que** la couche de couverture (18) est formée comme une pièce séparée (20) et est appliquée sur le piston (3).

8. Piston (3) selon l'une des revendications précédentes, **caractérisé en ce que** la couche de couverture (18) présente une ou plusieurs saillies (21) sur son côté tourné vers le piston (3) pour insertion par complémentarité de forme dans des évidements correspondants (22) dans le côté avant du piston (3).

9. Piston (3) selon la revendication 8, **caractérisé en ce que**, par rapport à une section transversale longitudinale à travers le piston (3), une saillie (21) et un évidement associé (22) sont agencés symétriquement ou sont traversés centralement par l'axe longitudinal (x).

10. Piston (3) selon l'une des revendications précédentes, **caractérisé en ce que** le piston (3) présente une couche de couverture (18) d'épaisseur (d) constante sur toute sa surface latérale (19), à l'exception de la zone du premier bourrelet d'étanchéité (15).

11. Piston (3) selon la revendication 10, **caractérisé en ce que** l'épaisseur constante (d) est de 0,3 à 3 µm.
